# FASCICULE DE BREVET EUROPEEN

(11) **EP 2 928 544 B1**
(45) Date de publication et mention de la délivrance du brevet: **24.08.2016**
(21) Numéro de dépôt: 13799048.7
(22) Date de dépôt: 02.12.2013
(51) Int. Cl.: C12N 13/00

(54) **DISPOSITIF D'APPLICATION DE CHAMP ELECTROMAGNETIQUE SUR UN ECHANTILLON BIOLOGIQUE**
VORRICHTUNG ZUR ANWENDUNG EINES ELEKTROMAGNETISCHEN FELDES AUF EINE BIOLOGISCHE PROBE
DEVICE FOR APPLYING AN ELECTROMAGNETIC FIELD TO A BIOLOGICAL SAMPLE

(30) Priorité: 04.12.2012 FR 1261622
(43) Date de publication de la demande: 14.10.2015
(73) Titulaire: Commissariat à l'Énergie Atomique et aux Énergies Alternatives, 75015 Paris (FR)
(72) Inventeur: VEZINET, René, F-46500 Bio (FR); CROIZER, Mathieu, F-19000 Brive (FR); DIOT, Jean-Christophe, F-46500 Gramat (FR); CATRAIN, Alexandre, F-46300 Le Vigan (FR)
(74) Mandataire: Brevalex
(86) Numéro de dépôt international: PCT/EP2013/075272
(87) Numéro de publication internationale: WO 2014/086725

(56) Documents cités:
- CAMP J T ET AL: "Bioelectric studies with subnanosecond pulsed electric fields", PULSED POWER CONFERENCE, 2009. PPC '09. IEEE, IEEE, PISCATAWAY, NJ, USA, 28 juin 2009 (2009-06-28), pages 876-879, XP031615053, ISBN: 978-1-4244-4064-1 cité dans la demande
- HEEREN T ET AL: "250 kV sub-nanosecond pulse generator with adjustable pulse-width", IEEE TRANSACTIONS ON DIELECTRICS AND ELECTRICAL INSULATION, IEEE SERVICE CENTER, PISCATAWAY, NJ, US, vol. 14, no. 4, août 2007 (2007-08), pages 884-888, XP011381654, ISSN: 1070-9878, DOI: 10.1109/TDEI.2007.4286520
- SCHOENBACH K H ET AL: "The Effect of Intense Subnanosecond Electrical Pulses on Biological Cells", IEEE TRANSACTIONS ON PLASMA SCIENCE, IEEE SERVICE CENTER, PISCATAWAY, NJ, US, vol. 36, no. 2, avril 2008 (2008-04), pages 414-422, XP011206838, ISSN: 0093-3813
- AUDE SILVE ET AL: "Nanosecond-Duration Electric Pulse Delivery In Vitro and In Vivo: Experimental Considerations", IEEE TRANSACTIONS ON INSTRUMENTATION AND MEASUREMENT, IEEE SERVICE CENTER, PISCATAWAY, NJ, US, vol. 61, no. 7, juillet 2012 (2012-07), pages 1945-1954, XP011445957, ISSN: 0018-9456, DOI: 10.1109/TIM.2012.2182861

## Description

### Domaine technique et art antérieur

L'invention concerne un dispositif d'application de champ électromagnétique sur un échantillon biologique.

Le domaine de l'invention est celui du bio-électromagnétisme qui a pour objet d'étudier les effets des champs électromagnétiques sur le vivant.

Les études conduites dans le cadre du bio-électromagnétisme nécessitent des systèmes expérimentaux aptes à exposer divers organismes biologiques, de tailles et de natures variées, aux champs électromagnétiques.

Les amplitudes des champs électromagnétiques peuvent être élevées (plusieurs dizaines de kV/cm) et le contenu spectral des signaux s'étend du continu aux très hautes fréquences (au-delà du GHz).

A ce jour, une grande variété de techniques existent pour l'application des champs électromagnétiques comme, par exemple :
- les systèmes d'exposition de type « ondes planes » ;
- les chambres réverbérantes ;
- les guides d'ondes ;
- la cellule fil-plaque ;
- la ligne de transmission radiale ;
- les cellules de Crawford et les cellules TEM (TEM pour « Transverse ElectroMagnétique ») ;
- le dispositif à résistance de charge coaxiale divulgué dans le document « BIOELECTRICAL STUDIES WITH SUBNANOSECOND PULSED ELECTRIC FIELDS » (J.Thomas Camp, Shu Xiao, Stephen J. Beebe, Peter F. Blackmore, and Karl H. Schoenbach ; Frank Reidy Research Center for Bioelectrics, Old Dominion University, Norfolk, VA*).*

Les principales caractéristiques et inconvénients des techniques de l'art antérieur vont être exposés ci-dessous.

Les systèmes d'exposition de type « ondes planes » utilisent des antennes rayonnantes qui doivent être placées dans des chambres anéchoïdes, lesquelles sont des installations coûteuses difficilement implantables dans un laboratoire de biologie. Ceci présente un réel inconvénient.

Les chambres réverbérantes fonctionnent exclusivement en régime sinusoïdal. Il n'est donc pas possible d'utiliser des impulsions, ce qui est un inconvénient. Par ailleurs, leur installation est souvent très délicate et aboutit à une structure encombrante.

Les guides d'ondes fonctionnent également exclusivement en régime sinusoïdal (pas d'impulsion) et leurs dimensions dépendent des fréquences utilisées.

La cellule fil-plaque utilise une antenne fil-plaque monopolaire et fonctionne exclusivement en régime sinusoïdal (pas d'impulsion) sur une faible largeur de bande.

La ligne de transmission radiale utilise une antenne conique centrale qui génère une onde transverse électro-magnétique. La bande passante de ce dispositif est également limitée.

Les cellules de Crawford (cellules fermées) et les cellules TEM (cellules ouvertes) sont des lignes de transmission de forme rectangulaire. Ces cellules sont capables de générer des champs électriques et magnétiques uniformes et perpendiculaires entre eux (mode TEM) dans la zone de test. Elles sont aptes à propager un courant continu et, de ce fait, sont bien adaptées à l'utilisation de signaux transitoires monopolaires ou bipolaires. Leur utilisation est cependant limitée en terme de tension maximale admissible et de compromis bande passante/dimensions utiles. La transition géométrique coaxial/biplaque ou coaxial/triplaque est complexe à réaliser pour les hautes fréquences, particulièrement avec des tensions élevées. Par ailleurs, la présence de l'objet sous test est - de fait - un obstacle qui est source de déformation du champ électromagnétique dans la zone de test.

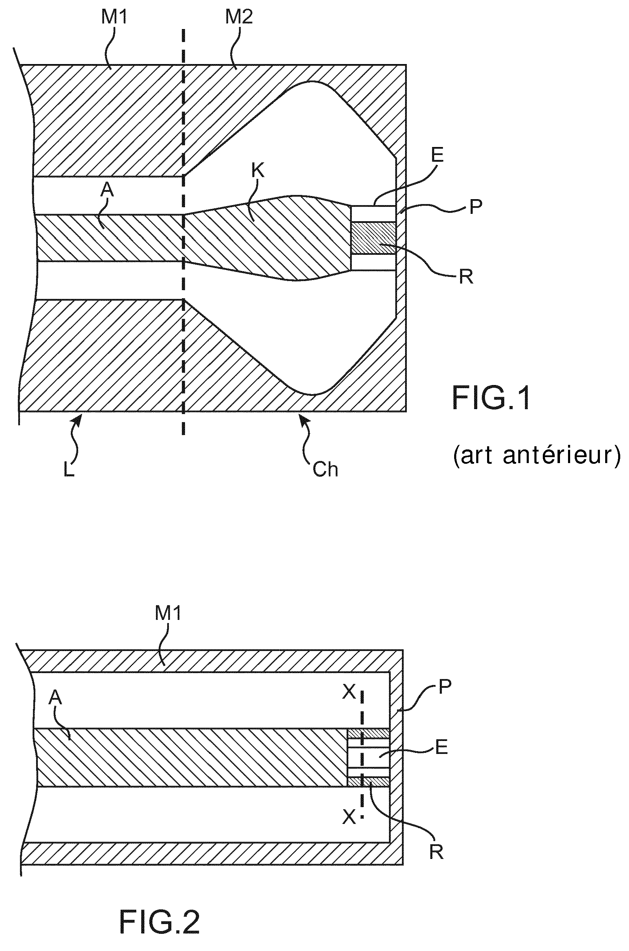

Le dispositif à résistance de charge coaxiale divulgué dans le document « BIOELECTRICAL STUDIES WITH SUBNANOSECOND PULSED ELECTRIC FIELDS » est proposé pour les applications in vitro dans lesquelles des impulsions de durées inférieures à la nano-seconde sont appliquées à des suspensions aqueuses. Ce montage est représenté en figure 1. Il est constitué par une ligne coaxiale L fermée à son extrémité par une charge Ch.

La ligne coaxiale L véhicule l'onde électromagnétique jusqu'à la charge Ch. La ligne L comprend un conducteur central A et un conducteur périphérique de masse M1. L'impédance de la charge Ch est adaptée à l'impédance de la ligne coaxiale L afin que l'onde incidente qui atteint la charge ne soit pas réfléchie. La charge Ch comprend un conducteur central K qui prolonge le conducteur central A de la ligne L et un conducteur périphérique de masse M2 qui prolonge le conducteur périphérique de masse M1. Le conducteur périphérique de masse M2 se referme par une paroi P formée dans un plan de section droite perpendiculaire à l'axe du coaxial. Une résistance R a une première borne électriquement reliée à l'extrémité du conducteur central K et une deuxième borne électriquement reliée à la paroi P du conducteur périphérique de masse M2. Alors que le conducteur central A a un diamètre constant tout le long de la ligne L, le diamètre du conducteur central K s'élargit puis se rétrécit entre le conducteur central A et la première borne de la résistance R. De même, alors que la distance qui sépare le conducteur périphérique de masse M1 du conducteur central A reste constante tout le long de la ligne L, la distance qui sépare le conducteur périphérique de masse M2 du conducteur K s'élargit fortement puis se rétrécit jusqu'à la paroi P qui ferme la charge Ch. La résistance R est alignée avec les conducteurs centraux A et K. L'échantillon E à tester est placé entre l'extrémité du conducteur coaxial K et la paroi P et entoure la résistance R.

Un tel montage est d'un usinage complexe de par les variations de côtes qu'il impose. Par ailleurs, l'échantillon E entourant la résistance R, il est nécessaire de prévoir des échantillons dont la partie centrale est évidée. Ceci représente également un autre inconvénient, car cela impose de réelles contraintes au niveau de la géométrie des échantillons.

L'invention ne présente pas ces inconvénients.

### Exposé de l'invention

En effet, l'invention concerne un dispositif d'application de champ électromagnétique sur un échantillon de matière biologique, le dispositif comprenant une structure électrique coaxiale ayant un conducteur central et un conducteur de masse qui entoure le conducteur central, une charge constituée d'une résistance électrique et de l'échantillon de matière biologique étant positionnée entre une extrémité du conducteur central et une paroi conductrice qui prolonge le conducteur de masse dans un plan sensiblement perpendiculaire à l'axe du conducteur central, la résistance électrique ayant une première extrémité reliée au conducteur central et une deuxième extrémité reliée à la paroi conductrice. La résistance électrique définit un volume intérieur dans lequel l'échantillon de matière biologique est placé.

Selon un premier mode de réalisation de l'invention, la résistance est constituée d'un ensemble de tubes résistifs pleins en parallèle qui définissent le volume intérieur dans lequel l'échantillon de matière biologique est placé.

Selon un deuxième mode de réalisation de l'invention, la résistance R est constituée d'un cylindre creux qui définit le volume intérieur dans lequel l'échantillon de matière biologique est placé.

### Brève description des figures

D'autres caractéristiques et avantages de l'invention apparaîtront à la lumière de la description qui va suivre, faite en référence aux figures jointes, parmi lesquelles :
- la figure 1, déjà décrite, représente un dispositif à résistance de charge coaxiale divulgué dans le document « BIOELECTRICAL STUDIES WITH SUBNANOSECOND PULSED ELECTRIC FIELDS »;
- la figure 2 représente un dispositif apte à appliquer un champ électromagnétique sur un échantillon biologique selon l'invention ;
- les figures 3A et 3B représentent deux variantes d'une vue en coupe transversale d'un dispositif de l'invention;
- les figures 4A et 4B représentent des modifications du dispositif représenté en figure 2;
- la figure 5 représente un premier perfectionnement du dispositif de l'invention ;
- les figures 6A-6D représentent différentes variantes du dispositif de l'invention ;
- les figures 7 et 8 illustrent des résultats de simulation de la variation en fonction du temps de l'amplitude du champ électrique qui est obtenue à l'aide d'un dispositif de l'invention, respectivement dans le cas d'un échantillon à vide et dans le cas d'un échantillon rempli d'eau ;
- la figure 9 illustre une localisation de points du dispositif de l'invention où la simulation des figures 7 et 8 est effectuée ;
- la figure 10 illustre un deuxième perfectionnement du dispositif de l'invention.

Sur toutes les figures, les mêmes références désignent les mêmes éléments.

### Exposé détaillé de modes de réalisation de l'invention

La figure 2 représente un dispositif apte à appliquer un champ électromagnétique sur un échantillon biologique selon l'invention.

Le dispositif comprend un câble coaxial fermé par une charge. Le câble coaxial est constitué d'un conducteur central A de diamètre constant et d'un conducteur périphérique de masse M1 dont la distance au conducteur central demeure constante jusqu'à l'extrémité du conducteur central. La charge est positionnée à l'extrémité du conducteur central. Elle est constituée d'une résistance R en forme de tube cylindrique creux et d'un échantillon E de matière biologique positionné à l'intérieur du tube cylindrique creux que forme la résistance. Le tube cylindrique creux a préférentiellement un diamètre extérieur identique au diamètre du conducteur central A et prolonge celui-ci jusqu'à une paroi conductrice plane P formée dans un plan de section droite perpendiculaire à l'axe du coaxial et qui ferme le conducteur périphérique de masse M1. L'échantillon E de matière biologique a une première face au contact de l'extrémité du conducteur central et une deuxième face, opposée à la première face, au contact de la paroi P. L'échantillon E de matière biologique est un solide ou liquide. Dans le cas où l'échantillon est solide, il peut être placé tel quel à l'intérieur du tube que forme la résistance R. Dans le cas où l'échantillon est liquide, il est placé dans un tube cylindrique creux fait d'une matière électriquement isolante. Dans tous les cas, la matière biologique solide ou liquide est au contact de l'extrémité du conducteur central A et de la paroi conductrice P.

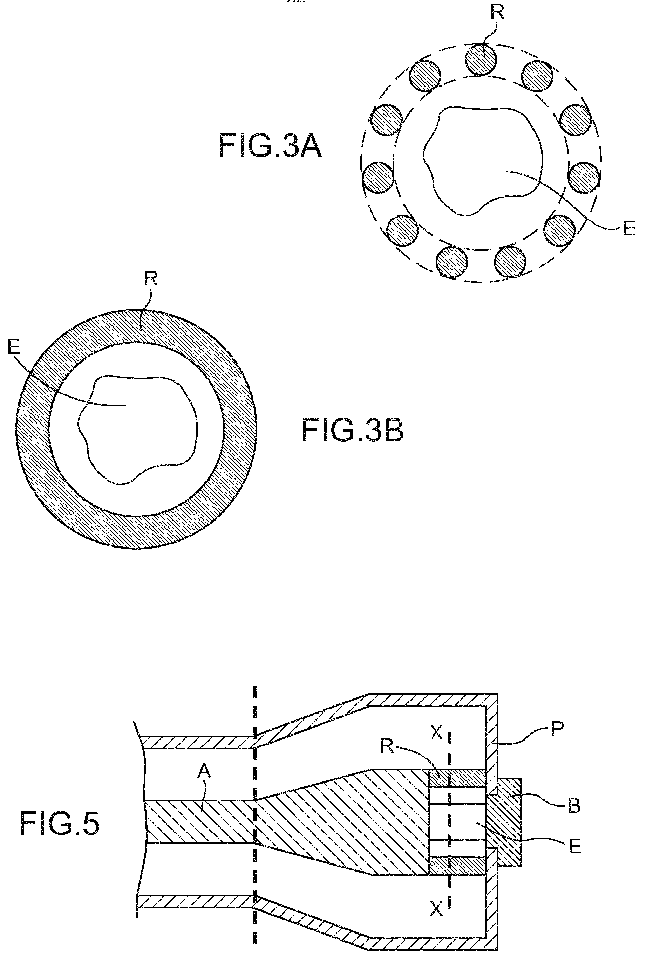

Les figures 3A et 3B représentent deux variantes d'une vue en coupe transversale selon l'axe XX d'un dispositif de l'invention.

Comme cela est illustré sur les figures 3A et 3B, la résistance R est composée, par exemple, d'un ensemble de tubes cylindriques résistifs pleins en parallèle (figure 3A) ou d'un tube résistif cylindrique creux unique (cf. figure 3B) dans lequel l'échantillon biologique vient se loger. De façon avantageuse, l'échantillon de matière biologique E ne présente pas d'évidement central. Dans la configuration géométrique du dispositif de l'invention, le champ électrique qui est présent dans l'espace que la résistance entoure est régi par la tension aux bornes de la résistance. Il s'ensuit que l'amplitude de ce champ est d'une grande homogénéité. Avantageusement, c'est dans cette zone que l'échantillon est placé conformément à l'invention. L'ensemble du volume de l'échantillon est ainsi soumis à un champ électrique ayant une amplitude d'une grande homogénéité.

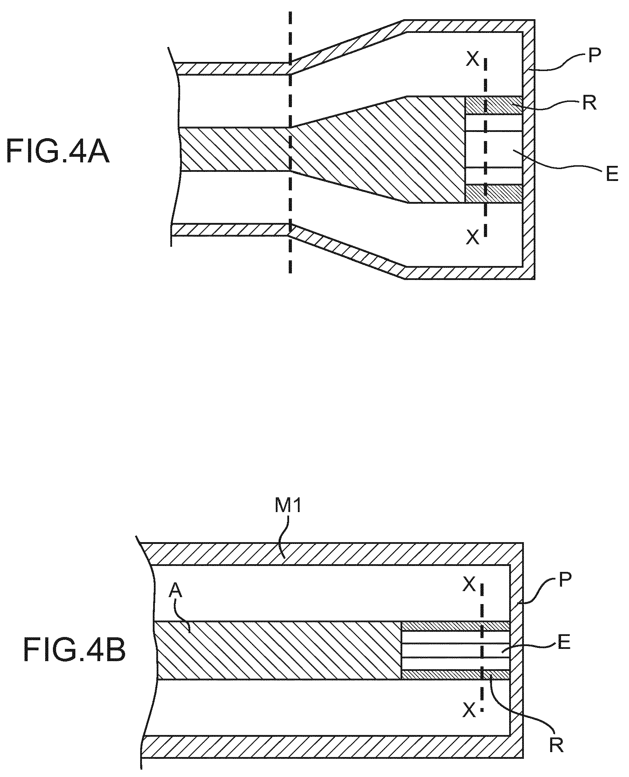

Les figures 4A et 4B représentent des modifications du dispositif représenté en figure 2.

La figure 4A illustre le cas où le coaxial s'élargit à son extrémité afin de permettre le montage d'échantillons plus volumineux. Le montage de l'échantillon est par ailleurs identique au montage du premier mode de réalisation de l'invention. Une résistance de charge entoure l'échantillon, la résistance de charge étant formée d'une pluralité d'un ensemble de tubes résistifs pleins en parallèle (figure 3A) ou d'un tube résistif cylindrique creux unique (figure 3B). La résistance de charge relie électriquement le conducteur central du coaxial à une paroi conductrice plane P qui prolonge le conducteur de masse et qui est formée dans un plan de section droite perpendiculaire à l'axe du coaxial. La résistance de charge régit ainsi localement la direction du champ électrique qui est en conséquence axial dans tout le volume intérieur à la charge résistive. Avantageusement, c'est dans ce volume intérieur que l'échantillon est placé conformément à l'invention.

La figure 4B illustre le cas où l'échantillon E est sensiblement plus long que dans le cas de la figure 2, toutes choses étant égales par ailleurs. Dans ce cas, aux fins d'adaptation d'impédance, le conducteur de masse M1 se rapproche de la résistance R avant d'atteindre la paroi P.

La figure 5 représente un premier perfectionnement du dispositif de l'invention. Selon ce perfectionnement, un bouchon ou une trappe amovible B est placé à l'extrémité du dispositif afin de pouvoir accéder au volume utile de l'échantillon. La paroi P est alors percée d'une ouverture que referme le bouchon ou la trappe. Le bouchon ou la trappe est une pièce électriquement conductrice, par exemple en métal. Le bouchon peut être massif ou ajouré. Dans ce dernier cas, il est traversé, par exemple, par des orifices de remplissage ou de purge.

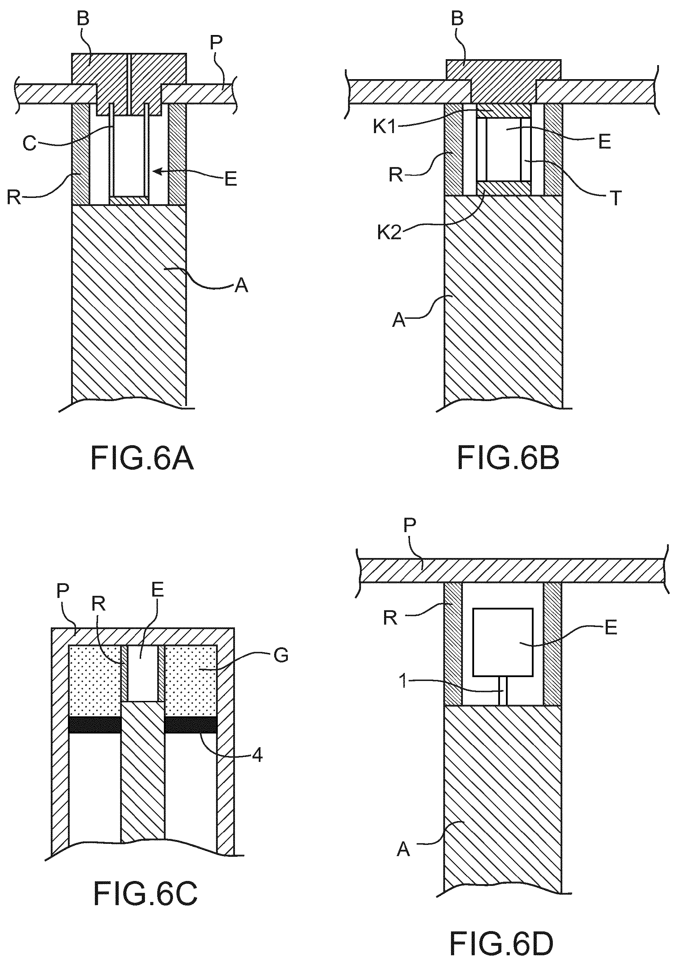

Les figures 6A-6D représentent différentes variantes du dispositif de l'invention.

La figure 6A correspond à un cas où le bouchon B est solidaire de l'échantillon E. La matière biologique, solide ou liquide, est alors placée dans une cuvette C qui est enfichée dans le bouchon B. La cuvette C a une paroi latérale faite d'un matériau électriquement isolant et un fond fait d'un matériau électriquement conducteur qui est en contact avec l'extrémité du conducteur central. La matière biologique présente dans la cuvette C est en contact avec le bouchon et avec le fond de la cuvette. Il est alors avantageusement possible de changer très simplement d'échantillon en retirant le bouchon et en plaçant un nouveau bouchon ou le même bouchon équipé d'un autre échantillon. Le contact entre l'extrémité du conducteur central et le fond de la cuvette C est de préférence un contact souple.

La figure 6B représente le cas où la matière biologique est contenue dans une enveloppe amovible constituée d'un tube cylindrique creux T fait de matière électriquement isolante fermée par deux éléments plats électriquement conducteurs K1, K2. Les éléments plats électriquement conducteurs K1, K2 sont en contact respectif avec un bouchon électriquement conducteur B et avec l'extrémité du conducteur central A. Le bouchon électriquement conducteur B est placé dans la paroi P. Il est alors avantageusement possible de changer d'échantillon en retirant le bouchon B.

La figure 6C représente le cas où, pour améliorer les performances en tension, un isolant liquide (par exemple de l'huile au Perfluoropolyéther) ou gazeux G (par exemple du gaz SF6) immerge la résistance R et l'échantillon de matière biologique. Une paroi électriquement isolante 4 sensiblement parallèle à la paroi conductrice P est alors placée entre le conducteur central A et le conducteur de masse M1 pour définir le volume qui contient la résistance R, l'échantillon E et le gaz ou le liquide. Ce mode de réalisation avec l'invention est compatible avec la présence d'un bouchon dans la paroi P (non représenté sur la figure 6C).

La figure 6D représente le cas où l'échantillon de matière biologique est un objet solide ou complexe OS (par exemple boîte de Petri, animal, etc.) qui est électriquement isolé du conducteur central A et de la paroi conductrice P. L'échantillon E est alors placé sur un pilier 1 fait dans un matériau électriquement isolant. Ce mode de réalisation de l'invention est également compatible avec la présence d'un bouchon B dans la paroi P (non représenté sur la figure).

Dans tous les cas mentionnés ci-dessus, une utilisation en haute tension, par exemple une tension de 25kV, conduit à un choix particulier des matériaux électriquement isolants utilisés et du matériau résistif qui constitue la résistance R, ainsi qu'à un choix particulier de géométries (par exemple, évitement des géométries à points triples). A titre d'exemple non limitatif, le matériau électriquement isolant utilisé pour les tubes C et T mentionnés précédemment peut être, par exemple, du polypropylène, et le matériau résistif de la résistance R peut être, par exemple, une céramique chargée de carbone.

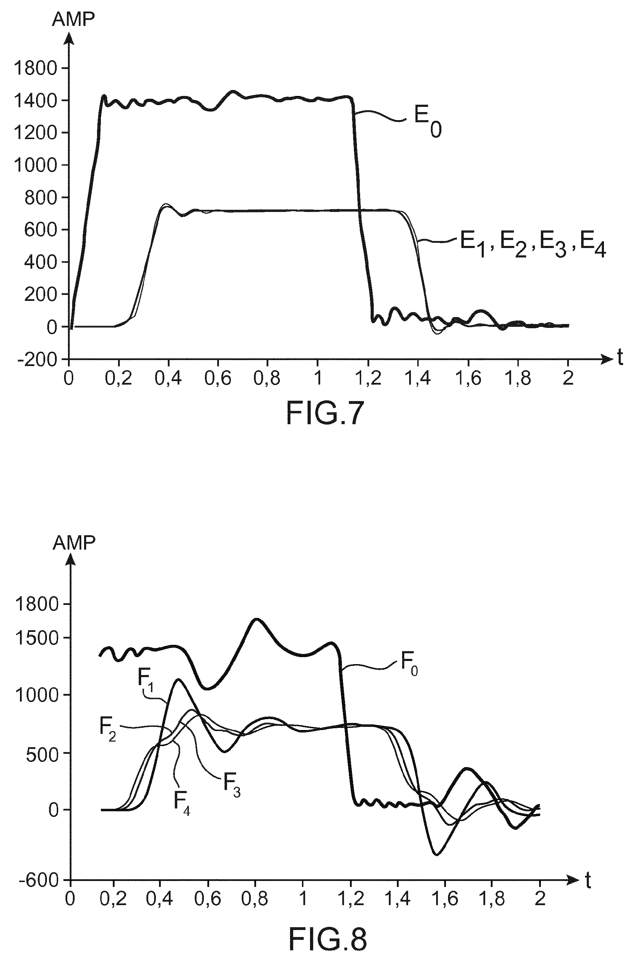

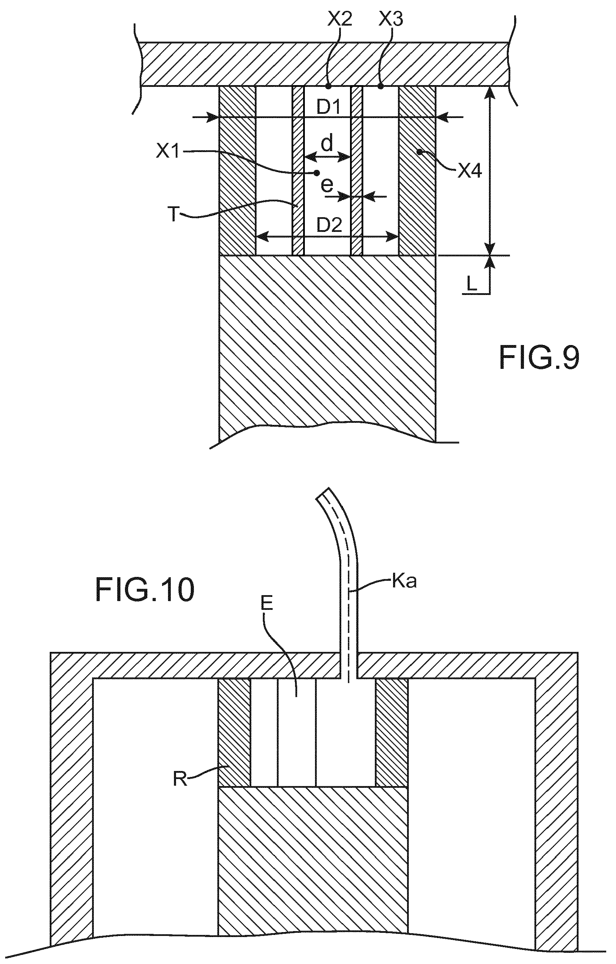

La figure 7 illustre des résultats de simulation de la variation en fonction du temps de l'amplitude AMP du champ électrique qui est obtenu, à vide, à l'aide d'un dispositif conforme à celui de la figure 2 et dont une vue de détail est donnée en référence à la figure 9. La figure 8 illustre les résultats obtenus, pour la même structure, avec un échantillon d'eau. L'amplitude AMP est exprimée en V/m et le temps t est exprimé en nanoseconde.

La résistance R est une résistance de 50Ω réalisée par un cylindre creux massif ayant une longueur L égale à 10mm, un diamètre extérieur D1 égal à 20mm et un diamètre intérieur D2 égal à 14mm. Le tube cylindrique creux T susceptible de contenir le liquide biologique présente un diamètre interne d de 4mm et une épaisseur e de 1mm. La colonne de liquide biologique susceptible d'être soumise à un champ électromagnétique a donc un diamètre de 4mm et une longueur de 10mm. Le câble coaxial d'impédance caractéristique égale à 50Ω est excité par une impulsion trapézoïdale de durée 1ns, de temps de montée 100ps et d'amplitude 7V.

Sur la figure 7, la courbe E₀ représente l'amplitude du champ électrique à l'entrée du câble coaxial (impulsion d'entrée) et les courbes E₁, E₂, E₃ et E₄ représentent, respectivement :
- l'amplitude du champ électrique en un point X1 situé dans le volume destiné à contenir la matière biologique (cf. figure 9),
- l'amplitude du champ électrique en un point X2 situé sur la face de la paroi P susceptible d'être en contact avec la matière biologique,
- l'amplitude du champ électrique en un point X3 situé à l'extérieur de l'espace intérieur défini par la résistance R, sur la face de la paroi P située à l'opposé du conducteur central,
- l'amplitude du champ électrique en un pont X4 situé à l'intérieur de la résistance R.

Il peut être constaté que, à vide, les courbes E₁, E₂, E₃ et E₄ sont avantageusement confondues.

Sur la figure 8, la courbe F₀ représente l'amplitude du champ électrique à l'entrée du câble coaxial (impulsion d'entrée) et les courbes F₁, F₂, F₃ et F₄ représentent, respectivement :
- l'amplitude du champ électrique au point X1,
- l'amplitude du champ électrique au point X2,
- l'amplitude du champ électrique au point X3,
- l'amplitude du champ électrique au point X4.

Les courbes F2 et F3 sont avantageusement confondues et, si les autres courbes ne se superposent pas parfaitement, il apparaît clairement que l'homogénéité de l'amplitude du champ électrique demeure excellente.

La figure 10 illustre un deuxième perfectionnement du dispositif de l'invention.

Selon ce deuxième perfectionnement, une antenne est placée dans la paroi P pour prélever les valeurs d'amplitude du champ électrique au point X3 défini précédemment. Cette antenne est réalisée, par exemple, à l'aide d'un câble coaxial Ka. En effet, en référence à la figure 8, il est possible de constater que la mesure de l'amplitude du champ électrique au point X3 est parfaitement représentative d'une mesure du champ électrique au point X2 et quasiment représentative d'une mesure du champ électrique au point X1. Il est ainsi possible, lors d'une manipulation, de surveiller l'amplitude du champ électrique qui est appliqué à l'échantillon biologique sur la base d'une mesure réalisée au point X3. Une telle surveillance n'est pas possible avec les dispositifs de l'art antérieur et, particulièrement, avec le dispositif décrit en figure 1 qui présente une très forte dispersion de l'amplitude du champ au niveau de l'échantillon, une mesure effectuée en un premier point ne pouvant alors être représentative d'une mesure effectuée en un point voisin du premier point.

## Revendications

1. Dispositif d'application de champ électromagnétique sur un échantillon de matière biologique (E), le dispositif comprenant une structure électrique coaxiale ayant un conducteur central (A) et un conducteur de masse (M1) qui entoure le conducteur central (A), une résistance électrique (R) positionnée entre une extrémité du conducteur central (A) et une paroi conductrice (P) qui prolonge le conducteur de masse (M1) dans un plan sensiblement perpendiculaire à l'axe du conducteur central (A), la résistance électrique (R) ayant une première extrémité reliée au conducteur central (A) et une deuxième extrémité reliée à la paroi conductrice (P), **caractérisé en ce que** ia résistance électrique (R) définit un volume intérieur dans lequel l'échantillon de matière biologique est placé (E).

2. Dispositif selon la revendication 1, dans lequel la résistance (R) est constituée d'un ensemble de tubes résistifs pleins en parallèle qui définissent le volume intérieur dans lequel l'échantillon de matière biologique (E) est placé.

3. Dispositif selon la revendication 1, dans lequel la résistance R est constituée d'un cylindre creux qui définit le volume intérieur dans lequel l'échantillon de matière biologique (E) est placé.

4. Dispositif selon l'une quelconque des revendications précédentes, dans lequel un bouchon ou une trappe amovible (B) est placé dans la paroi électriquement conductrice pour autoriser l'accès à l'échantillon de matière biologique (E).

5. Dispositif selon la revendication 4, dans lequel la matière biologique est contenue dans une cuvette (C) qui est enfichée dans le bouchon ou la trappe, la cuvette ayant une paroi latérale faite d'un matériau électriquement isolant et un fond fait d'un matériau électriquement conducteur au contact de l'extrémité du conducteur central (A).

6. Dispositif selon la revendication 4, dans lequel la matière biologique est contenue dans une enveloppe constituée d'un tube cylindrique creux (T) fait de matière électriquement isolante fermé par deux éléments plans électriquement conducteurs (K1, K2) en contact respectif avec le bouchon ou la trappe (B) et avec l'extrémité du conducteur central (A).

7. Dispositif selon l'une quelconque des revendications 1 à 3, dans lequel la matière biologique est contenue dans une enceinte constituée par une fraction de surface de la paroi conductrice (P), par une fraction de surface de l'extrémité du conducteur central (A) située en regard de la fraction de surface de la paroi conductrice (P) et par un tube cylindrique creux fait de matière électriquement isolante situé entre la paroi conductrice et l'extrémité du conducteur central.

8. Dispositif selon l'une quelconque des revendications 1 à 4, dans lequel la matière biologique est un corps solide.

9. Dispositif selon la revendication 8, dans lequel le corps solide est une boite de Pietri, un objet ou un animal.

10. Dispositif selon l'une quelconque des revendications précédentes, dans lequel une paroi électriquement isolante (4) placée entre le conducteur central (A) et le conducteur de masse (M1) définit un volume fermé qui contient la résistance électrique (R) et l'échantillon de matière biologique (E), un liquide ou un gaz électriquement isolant (G) baignant l'espace libre du volume fermé.

## Patentansprüche

1. Vorrichtung zur Anlegung eines elektromagnetischen Feldes an eine Probe aus biologischem Material (E), wobei die Vorrichtung Folgendes umfasst: einen koaxialen elektrischen Aufbau, welcher einen zentralen Leiter (A) und einen Erdungsleiter (M1) aufweist, welcher den zentralen Leiter (A) umgibt; weiter einen elektrischen Widerstand (R), welcher zwischen einem Ende des zentralen Leiters (A) und einer leitenden Wand (P), welche den Erdungsleiter (M1) in eine Ebene im Wesentlichen senkrecht zur Achse des zentralen Leiters (A) verlängert, angeordnet ist, wobei der elektrische Widerstand (R) ein erstes Ende, welches mit dem zentralen Leiter verbunden ist, sowie eine zweites Ende aufweist, welches mit der leitenden Wand (P) verbunden ist, **dadurch gekennzeichnet, dass** der elektrische Widerstand (R) einen Innenraum begrenzt, in welchem die Probe aus biologischem Material (E) angeordnet ist.

2. Vorrichtung nach Anspruch 1, in welcher der Widerstand (R) aus einer Gruppe von parallelen Widerstands-Vollrohren gebildet wird, welche den Innenraum begrenzen, in welchem die Probe aus biologischem Material (E) angeordnet ist.

3. Vorrichtung nach Anspruch 1, in welcher der Widerstand (R) aus einem Hohlzylinder gebildet ist, welcher den Innenraum begrenzt, in welchem die Probe aus biologischem Material (E) angeordnet ist.

4. Vorrichtung nach einem der vorhergehenden Ansprüche, in welcher ein Verschluss oder eine bewegliche Klappe bzw. Falltür (B) in der elektrisch leitenden Wand angeordnet ist, um den Zugriff auf die Probe aus biologischem Material (E) zu gestatten.

5. Vorrichtung nach Anspruch 4, in welcher das biologische Material in einer Schüssel (C) enthalten ist, welche auf den Verschluss oder die Klappe bzw. Falltür aufgesteckt wird, wobei die Schüssel eine aus einem elektrisch isolierenden Material hergestellte Seitenwand und einen aus einem elektrisch leitenden Material hergestellten Boden in Kontakt mit dem Ende des zentralen Leiters (A) aufweist.

6. Vorrichtung nach Anspruch 4, in welcher das biologische Material in einer Hülle enthalten ist, welche aus einem zylindrischen Hohlrohr (T) gebildet ist, das aus einem elektrisch isolierendem Material hergestellt ist, wobei das Hohlrohr durch zwei elektrisch leitende plane Elemente (K1, K2) geschlossen wird, welche jeweils mit dem Verschluss oder der Klappe bzw. Falltür (B) und mit dem Ende des zentralen Leiters (A) in Verbindung stehen.

7. Vorrichtung nach einem der Ansprüche 1 bis 3, in welcher das biologische Material in einem Gehäuse enthalten ist, welches aus einem Teil der Fläche der leitenden Wand (P), einem Teil der Fläche des Endes des zentralen Leiters (A), welcher gegenüber dem Teil der Fläche der leitenden Wand (P) angeordnet ist, sowie aus einem zylindrischen Hohlrohr gebildet ist, welches aus elektrisch isolierendem Material hergestellt ist und zwischen der leitenden Wand und dem Ende des zentralen Leiters angeordnet ist.

8. Vorrichtung nach einem der Ansprüche 1 bis 4, in welcher das biologische Material ein Festkörper ist.

9. Vorrichtung nach Anspruch 8, in welcher der Festkörper eine Petrischale, ein Gegenstand oder ein Tier ist.

10. Vorrichtung nach einem der vorhergehenden Ansprüche, in welcher eine zwischen dem zentralen Leiter (A) und dem Erdungsleiter (M1) angeordnete elektrisch isolierende Wand (4) ein geschlossenes Volumen begrenzt, welches den elektrischen Widerstand (R) und die Probe aus biologischem Material (E) umfasst, wobei eine Flüssigkeit oder ein elektrisch isolierendes Gas (G) den freien bzw. leeren Raum des geschlossenen Volumens durchfließt.

## Claims

1. A device for applying an electromagnetic field to a biological feedstock sample (E), the device comprising a coaxial electrical structure having a center conductor (A) and a ground conductor (M1) which surrounds the center conductor (A), an electrical resistor (R) positioned between one end of the center conductor (A) and a conductive wall (P) which extends the ground conductor (M1) into a plane substantially perpendicular to the axis of the center conductor (A), the electrical resistor (R) having a first end connected to the center conductor (A) and a second end connected to the conductive wall (P), **characterized in that** the electrical resistor (R) defines an internal volume wherein the biological feedstock sample is placed (E).

2. The device according to claim 1, wherein the resistor (R) consists of an assembly of parallel solid resistive tubes which define the internal volume wherein the biological feedstock sample (E) is placed.

3. The device according to claim 1, wherein the resistor (R) consists of a hollow cylinder which defines the internal volume wherein the biological feedstock sample (E) is placed.

4. The device according to any of the preceding claims, wherein a removable plug or trapdoor (B) is placed in the electrically conductive wall to allow access to the biological feedstock sample (E).

5. The device according to claim 4, wherein the biological feedstock is contained in a bowl (C) which is inserted in the plug or trapdoor, the bowl having a side wall made of an electrically insulating material and a bottom made of an electrically conductive material in contact with the end of the center conductor (A).

6. The device according to claim 4, wherein the biological feedstock is contained in a shell consisting of a hollow cylindrical tube (T) made of an electrically insulating material closed by two electrically conductive planar elements (K1, K2) in respective contact with the plug or trapdoor (B) and with the end of the center conductor (A).

7. The device according to any of claims 1 to 3, wherein the biological feedstock is contained in an enclosure consisting of a surface fraction of the conductive wall (P), by a surface fraction of the end of the center conductor (A) located facing the surface fraction of the conductive wall (P) and by a hollow cylindrical tube made of an electrically insulating material located between the conductive wall and the end of the center conductor.

8. The device according to any of claims 1 to 4, wherein the biological feedstock is a solid body.

9. The device according to claim 8, wherein the solid body is a Pietri dish, an object or an animal.

10. The device according to any of the preceding claims, wherein an electrically insulating wall (4) placed between the center conductor (A) and the ground conductor (M1) defines a closed volume which contains the resistor (R) and the biological feedstock sample (E), an electrically insulating liquid or gas (G) soaking the head space of the closed volume.
